**Europäisches Patentamt**

**European Patent Office**

**Office européen des brevets**

(19)

(11) Veröffentlichungsnummer: **0 019 104**
**A2**

(12)

# EUROPÄISCHE PATENTANMELDUNG

(21) Anmeldenummer: **80102159.3**

(22) Anmeldetag: **22.04.80**

(51) Int. Cl.³: **A 61 B 10/00**

(30) Priorität: **19.05.79 DE 7914565 U**

(43) Veröffentlichungstag der Anmeldung:
**26.11.80  Patentblatt  80 24**

(84) Benannte Vertragsstaaten:
**AT BE CH DE FR GB IT LI LU NL SE**

(71) Anmelder: **Intermedicat GmbH**
**Gerliswilstrasse 45**
**CH-6020 Emmenbrücke(CH)**

(72) Erfinder: **Dinter, Jürgen**
**Quergasse 11**
**D-3508 Melsungen(DE)**

(74) Vertreter: **Selting, Günther, Dipl.-Ing. et al,**
**Deichmannhaus am Hauptbahnhof**
**D-5000 Köln 1(DE)**

(54) **Biopsiekanüle zur Entnahme histologischer Gewebeproben.**

(57) Die Erfindung betrifft eine Biopsiekanüle zur Entnahme histologischer Gewebeproben aus dem menschlichen oder tierischen Organismus. Sie besteht aus einer Kanüle (11), deren Schneidkante (12) vorne angespitzt ist und die auf einer Probennadel (1) über eine Distanz verschiebbar ist, die mindestens der Länge einer Probenkerbe (2) entspricht, die hinter der Spitze der Probennadel (1) angeordnet ist und zusammen mit der Kanüle (11) eine Tasche zur Aufnahme der Gewebeproben bildet. Die Probenkerbe (2) der Probennadel (1) läuft am spitzenfernen Ende in einen relativ großen, von der Umfangslinie zum Probenkerbengrund (2) verlaufenden Radius aus und an ihrem spitzennahen Ende ist eine senkrecht zur Probennadelachse gerichtete Fläche eingeschliffen.

FIG. 5

EP 0 019 104 A2

-1-

Biopsiekanüle zur Entnahme histologischer Gewebeproben

---

Die Erfindung bezieht sich auf eine Biopsiekanüle zur Entnahme histologischer Gewebeproben aus dem menschlichen oder tierischen Organismus, bestehend aus einer Kanüle mit einer vorderen abgeschrägten Schneidkante, in der eine an ihrem vorderen Ende mit einer angeschliffenen Spitze versehene Probennadel angeordnet ist, wobei beide Teile relativ zueinander axial verschiebbar sind und die Probennadel hinter ihrer Spitze eine Probenkerbe aufweist, die zusammen mit der Kanüle eine Tasche zur Aufnahme der Gewebeproben bildet.

Eine bekannte Vorrichtung zur Entnahme von Gewebeproben zur histologischen Untersuchung nach "Vim-Silverman" besteht aus einer in einer Kanüle gleitbaren Probennadel, deren vorderes Ende in Längsrichtung gespalten ist, und deren beiden Spitzen harpunenspitzenartig hakenförmig gestaltet sind. Die Probennadel wird in geschlossenem Zustand in das zu untersuchende Gewebe eingeführt, wobei sich die beiden länglichen Spitzenteile auseinanderspreizen. Die anschließend über die beiden Spitzenteile vorgeschobene Kanüle führt diese zusammen und trennt das zwischen den hakenförmigen Ausbildungen befindliche und erfaßte Gewebe ab. Erfahrungsgemäß erhält man mit diesem Instrument eine geringe Gewebemenge, die zudem zum großen Teil gequetscht ist und anderweitig zerstörtes, zerrissenes Material enthält, das für eine histologische Untersuchung nur noch bedingt verwendbar ist. Zu diesem unbefriedigenden Ergebnis addiert sich die mechanische Instabilität der geteilten Probennadel, deren Spitzenteile bei dem vorgegebenen geringen Durchmesser wenig stabil sind und die bei den zur Probengewinnung erforderlichen Manipulationen durch die aufgebrachten Hebe- und Biegekräfte besonders im Bereich der Haken abbrechen. Es besteht also die Gefahr, daß Teile des Biopsieinstrumentes in dem zu untersuchenden Organ verbleiben.

Ein anderes bekanntes Biopsieinstrument ist in der DE-PS 1 817 555 beschrieben. Dieses besteht aus einer Kanüle mit einer vorderen abgeschrägten Schneidkante, in der eine an ihrem vorderen Ende mit einer angeschliffenen Spitze versehene Probennadel angeordnet ist, die hinter ihrer Schneidkante eine rechteckige Probenkerbe aufweist. Die Probennadel ist in der Kanüle axial verschiebbar angeordnet, und ihre Probenkerbe bildet zusammen mit der Kanüle eine Tasche zur Aufnahme von Gewebeproben. Die vordere abgeschrägte Schneidkante der Kanüle weist einen außenliegenden, dem abgeschrägten Kanülenmantel folgenden Schliff auf, der in einer angerundeten Spitze mündet. Die Probenkerbe der um einen vorbestimmten Weg im Innern der Kanüle verschiebbaren Probennadel befindet sich in ihrem vorderen Bereich, ist etwa 2 bis 3 cm lang und rechteckig scharfkantig in die Probennadel eingeschliffen. Bei eingeführtem Biopsieinstrument legt sich das umgebende Gewebe in die Probenkerbe und wird vom Schneidkantenschliff der darübergeschobenen Kanüle aus dem Gewebeverbund abgetrennt. Die 2 bis 3 cm lange Probenkerbe nimmt etwa 2/3 des Probennadeldurchmessers in der Höhe im Spitzenbereich der Probennadel ein. Ihre beiden begrenzenden Seiten werden durch je einen senkrecht zur Nadelachse stehenden

-3-

geraden Schliff gebildet. Die an der Probennadel angeschliffene Spitze erleichtert das Einstechen des Instrumentes bis zum Ort der Probenentnahme.

Obwohl diese Ausführungsform eine Verbesserung sowohl in der Handhabung als auch im Punktionserfolg mit sich bringt, sind einige Nachteile feststellbar, die sich unvorteilhaft auf den Gebrauch des Instrumentes auswirken. Da der Schneidkantenschliff der Kanüle am vorderen Ende abgerundet ausgebildet ist, und deshalb bei der Einführung in das zu untersuchende Organ das Gewebe unterläuft, muß zur Erfassung einer ausreichend oder maximal großen Gewebeprobe die Probennadel kräftig gegen das umgebende Gewebe gedrückt werden, um es in ausreichendem Maße in der Probenkerbe unterzubringen. Hierdurch wird die Führung des das Gewebe abtrennenden Schneidkantenschliffs der Kanüle etwas diffus, und das Gewebe wird vom Schliff mehr oder weniger beiseite gedrückt, so daß die Probenmenge gering und die Probe gezupft sein kann. Zusätzlich wirkt sich bei der Anpressung der Probennadel gegen das Gewebe nachteilig aus, daß bei großer Probenkerbe,die zur Gewinnung brauchbarer Proben erwünscht sein muß, die Festigkeit des an der Probenkerbe verbliebenen Werkstoffmaterials nicht ausreichen kann, um das aufgebrachte Biegemoment auszuhalten. Als Folge einer derartigen Beanspruchung kann die Probennadel an der spitzenfern gelegenen Kerbenkante abknicken oder abbrechen und damit den angestrebten Erfolg vereiteln.

Dieselbe Situation ergibt sich, wenn bei einer Punktion die über der Probennadel und damit über der Probenkerbe befindliche Kanüle bereits zurückgezogen ist und ihre stützende, schienende und versteifende Funktion für den Kerbenbereich entfällt. Ein durch weitere notwendige Manipulation erzeugtes zu hohes Biegemoment bringt auch hierbei die Probennadel in einen Zustand, in dem der verringerte Probennadelquerschnitt im Probenkerbenbereich der Belastung nicht Stand hält und der vordere labile Teil der Probennadel sich entweder bleibend verbiegt oder sogar abbricht.

Der Erfindung liegt die Aufgabe zugrunde, die Produktsicherheit einer Biopsiekanüle bzw. eines Biopsieinstrumentes ohne Probeneinbuße gegenüber herkömmlichen Entnahmebestecken zu erhöhen. Im einzelnen bedeutet dies, daß die Probennadel so ausgebildet werden soll, daß sie Biegekräfte ohne Beeinträchtigungen oder Schädigungen ihres Kerbenbereiches aufnehmen kann, und der Schneidkantenschliff der Hülse so vorzusehen ist, daß er in optimaler Weise die durch Biegekräfte im Festigkeitsgrenzbereich beanspruchte Probenkerbe beim Schneide- und Trennvorgang wirksam entlastet.

Diese Aufgabe wird dadurch gelöst, daß die Schneidkante der Kanüle vorne angespitzt und die Kanüle auf der Probennadel über eine mindestens der Länge der Probenkerbe entsprechende Distanz verschiebbar ist, und daß die Probenkerbe der Probennadel am spitzenfernen Ende in einen relativ großen, von der Umfangslinie zum Probenkerbengrund verlaufenden Radius ausläuft und an ihrem spitzennahen Ende eine senkrecht zur Probennadelachse gerichtete Fläche eingeschliffen ist.

Ein in dieser Weise ausgebildete Biopsiekanüle weist eine in der Schneidwirkung verbesserte Kanülenspitze und eine auf höhere Biegebeanspruchung belastbare knickfeste Probennadel auf. Die Formgebung der Probenkerbe gewährleistet darüber hinaus eine hinsichtlich des Punktionserfolges und der Probengröße zuverlässigere Funktionssicherheit. Die spitze Schneidkante der Kanüle zertrennt das Gewebe,in das das Biopsie-instrument eingeführt ist, bei ihrer Verschiebung in Richtung der Probennadelspitze mit einem scharfen Trennschnitt und das nachteilige Unterlaufen des Gewebes wird vermieden. Die exakt ausgeführte Spitze der Kanüle bewirkt, daß bei dem Schneidevorgang der Gewebeabtrennung die Kanülenbewegung ohne die Tendenz einer seitlichen Ab- oder Auslenkung erfolgt. Der Trennschnitt ist sauber und glatt, und die in der Probenkerbe eingeschlossene Gewebeprobe ist insgesamt für histologische Untersuchungen geeignet. Durch die Kombination einer spitzennahen zur Längsachse der Probennadel senkrecht verlaufenden Wand der Probenkerbe und einer spitzenfernen nach hinten schräg und gekrümmt auslaufenden Wand ergibt sich bei höheren Biegekräften eine knickfeste Stabilität, wobei das verfügbare Probenkerbenvolumen groß bleibt  und die Entnahme sehr großer Gewebeproben erlaubt.Da im spitzennahen Bereich der Probennadel nur geringe Biegekräfte zu erwarten sind, ist an dieser Stelle eine Querschnittsverstärkung nicht erforderlich, weshalb die Probenkerbenwand hier senkrecht zur Probennadelachse verlaufen kann, damit ihre obere Kante beim Auftreffen der Spitze der Schneidkante der Kanüle als Widerlager für das abzuschneidende Gewebe wirksam sein kann. Hauptsächlich greifen die Biegekräfte an dem dem Probennadelende bzw. dem Probennadelgriffansatz zugewandten Teil der Probenkerbe an. In dieser Zone ist der Probennadelquerschnitt durch den schrägen, abgerundeten Verlauf der hinteren Wand der Probennadel ausreichend verstärkt,um Hebelwirkungen ausreichenden Widerstand entgegenzusetzen. Wird die in das zu untersuchende Organ eingeführte Probennadel kräftig gegen das umgebende Gewebe gedrückt, damit eine möglichst große Probenmenge in die Probenkerbe eintritt, so ist die Knickfestigkeit der Probennadel im hinteren Bereich der Probenkerbe gewährleistet. Außerdem wird durch diesen Verlauf der hinteren
Probenkerbenwand das Volumen der Probenkerbe vergrößert.

Um eine bessere Fixierung der in die Probenkerbe eingedrückten Gewebeprobe vor und während des Trennvorganges
zu erreichen, kann die senkrecht zur Probennadelachse
gerichtete Fläche am Übergang zum Probenkerbengrund einen
konkaven (Hohl-) Schliff geringen Radius' aufweisen.

Zur Verbesserung der Schneidwirkung der Schneidkante der
Kanüle verläuft die Spitze vorteilhaft radiusfrei und
gerade, so daß keinerlei Ablenkung des zu zerteilenden
Gewebes erfolgt.

In der Zeichnung ist ein Ausführungsbeispiel der
Biopsiekanüle schematisch veranschaulicht.

Es zeigt:

   Fig. 1 eine Seitenansicht des vorderen Endes der Probennadel,
   Fig. 2 eine Draufsicht der Probennadel nach Figur 1,

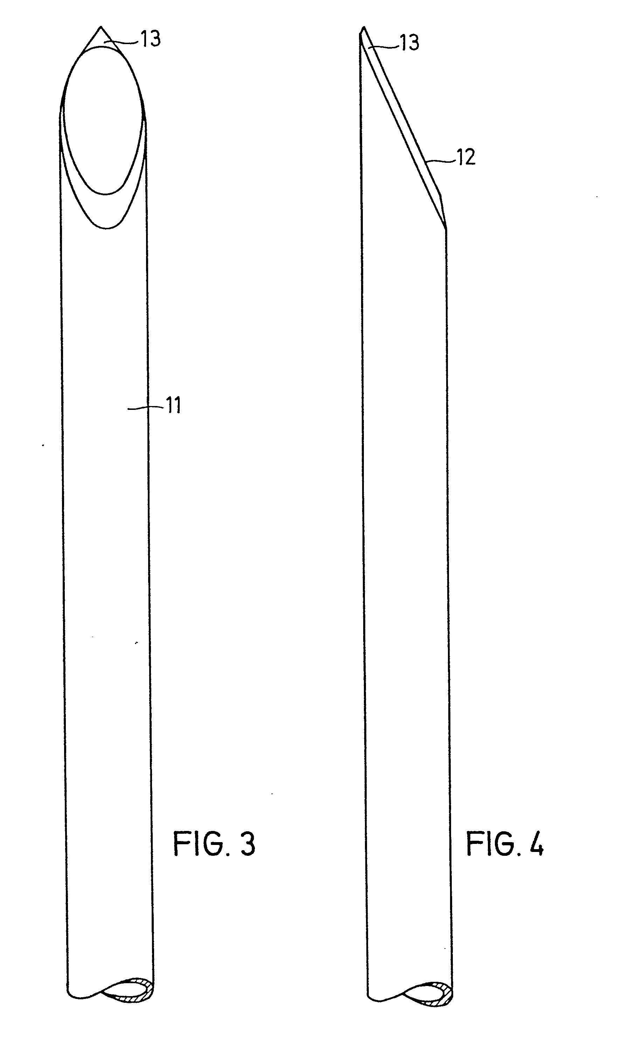

   Fig. 3 eine Draufsicht der Kanüle,
   Fig. 4 eine Ansicht der Kanüle in einer um 90$^{o}$ zu der
Darstellung der Figur 3 gedrehten Position, und
   Fig. 5 das vordere Ende der Kanüle mit in diese eingesteckter Probennadel im Zustand der Probengewinnung.

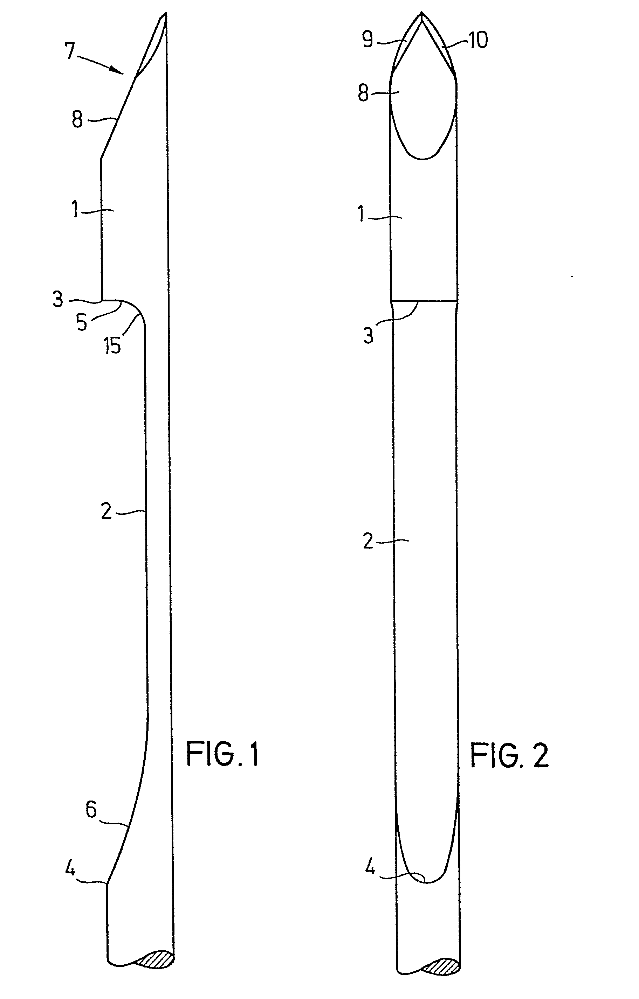

Gemäß Fig. 1 ist eine Probennadel 1 mit einer Probenkerbe
2 versehen, die von einer spitzennahen Wand 3 und einer
spitzenfernen Wand 4 begrenzt ist. Die Wand 3 ist als
zur Probennadellängsachse senkrechter Schliff 5 ausgeführt. An ihrem Übergang zum Grund der Probenkerbe 2 weist sie einen konkaven Schliff 15 geringen Radius' auf. Die Wand 4 läuft in einen relativ großen von der Umfangslinie zum Probenkerbengrund verlaufenden Radius (6) aus, d.h. sie hat einen gekrümmt schrägen Verlauf nach hinten. In der Zone zwischen dem Beginn der Krümmungen der vorderen Wand 3 und der hinteren Wand 4 ist der Probenkerbengrund eben und gerade.

Das vordere Ende 7 der Probennadel 1 ist durch einen schrägen Normalschliff 8 angespitzt und durch einen Facettenschliff mit Facetten 9 und 10 (Fig. 2) angeschärft.

Auf der Probennadel 1 ist eine Kanüle 11 axial verschiebbar angeordnet. Das vordere Ende der Kanüle ist ebenfalls abgeschrägt. Der abgeschrägte Rand weist einen an der Kontur angebrachten umlaufenden Schliff 12 auf, der in eine angeschliffene Spitze 13 mit einem zugespitzten Ende übergeht.

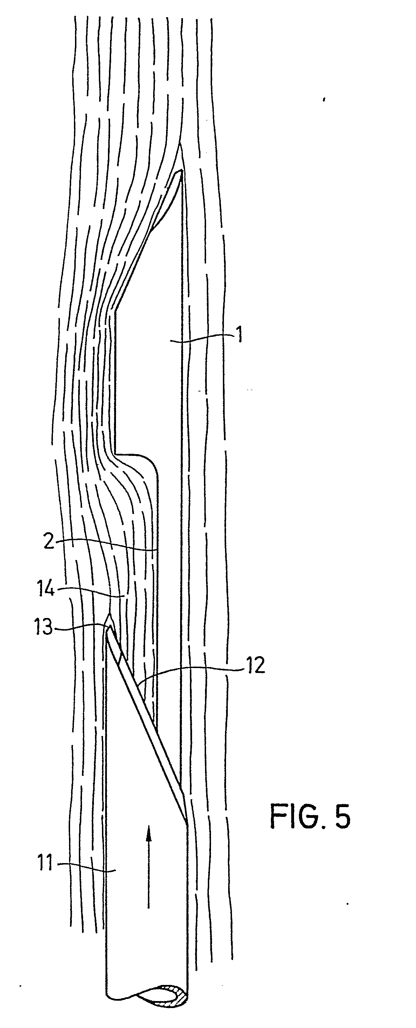

Fig. 5 ist die Darstellung des Zustandes der Probengewinnung. Hierbei wird die Kanüle 11 in Pfeilrichtung relativ zur statisch gehaltenen Probennadel 1 bewegt, und es wird die in die Probenkerbe 2 eingedrückte Gewebemasse 14 durch den Schliff 12 und die Spitze 13 der Kanüle 11 getrennt.

A N S P R Ü C H E

1. Biopsiekanüle zur Entnahme histologischer Gewebeproben aus dem menschlichen oder tierischen Organismus,
bestehend aus einer Kanüle mit einer vorderen abgeschrägten Schneidkante, in der eine an ihrem vorderen Ende mit
einer angeschliffenen Spitze versehene Probennadel angeordnet ist, wobei beide Teile relativ zueinander axial
verschiebbar sind und die Probennadel hinter ihrer
Spitze eine Probenkerbe aufweist, die zusammen mit der
Kanüle eine Tasche zur Aufnahme der Gewebeproben bildet,
d a d u r c h   g e k e n n z e i c h n e t,   daß
die Schneidkante (12) der Kanüle (11) vorne angespitzt
und die Kanüle (11) auf der Probennadel (1) über eine
mindestens der Länge der Probenkerbe (2) entsprechende
Distanz verschiebbar ist, und daß die Probenkerbe (2)
der Probennadel (1) am spitzenfernen Ende in einen relativ großen, von der Umfangslinie (4) zum Probenkerbengrund (2) verlaufenden Radius (6) ausläuft und an ihrem
spitzennahen Ende eine senkrecht zur Probennadelachse
gerichtete Fläche (3,5) eingeschliffen ist.

2. Biopsiekanüle nach Anspruch 1,   d a d u r c h
g e k e n n z e i c h n e t,   daß die senkrecht
zur Probennadelachse gerichtete Fläche (4) am Übergang zum Probenkerbengrund (2) einen konkaven (Hohl-)
Schliff (15) geringen Radius' aufweist.

3. Biopsiekanüle nach Anspruch 1,
d a d u r c h   g e k e n n z e i c h n e t,   daß
die Spitze (13) der Schneidkante (12) der Kanüle (11)
radiusfrei ausläuft

FIG.1

FIG. 2

13

11

**FIG. 3**

13

12

**FIG. 4**

FIG. 5